# EUROPEAN PATENT APPLICATION

(11) **EP 2 216 037 A1**
(43) Date of publication of application: **11.08.2010**
(21) Application number: 08857521.2
(22) Date of filing: 05.12.2008
(51) Int. Cl.: A61K 36/47, A23L 1/30, A23L 2/52, A61K 8/49, A61K 8/97, A61K 31/353, A61K 38/00, A61P 1/02, A61P 31/04, A61Q 11/00, C07D 311/32

(54) **PERIODONTAL BACTERIAL GROWTH INHIBITOR, ORAL HYGIENE PRODUCT, AND FOOD AND DRINK**

(30) Priority: 05.12.2007 JP 2007314796
(71) Applicant: POKKA CORPORATION, Nagoya-shi Aichi 460-8415 (JP)
(72) Inventor: GOTO, Takaki, Nagoya-shi Aichi 460-8415 (JP); FUKUMOTO, Syuichi, Nagoya-shi Aichi 460-8415 (JP)
(74) Representative: Viering, Jentschura & Partner
(86) International application number: PCT/JP2008/072134
(87) International publication number: WO 2009/072594

(57) **Abstract**

A periodontal bacterial growth inhibitor contains as an active ingredient a *Macaranga tanarius* extract extracted from *Macaranga tanarius* with an extraction solvent including at least an organic solvent. Alternatively, the periodontal bacterial growth inhibitor contains as an active ingredient at least one selected from nymphaeol-A, nymphaeol-B, and nymphaeol-C. The periodontal bacterial growth inhibitor is used by being blended to, for example, an oral hygiene product or a food and drink.

## Description

### TECHNICAL FIELD

The present invention relates to a periodontal bacterial growth inhibitor, and relates to an oral hygiene product and a food and drink that contain the inhibitor.

### BACKGROUND ART

Periodontal disease developed by periodontal bacterial infection is roughly classified into gingivitis, periodontitis, and occlusal trauma. Recently, it has been revealed that infectious diseases caused by periodontal bacteria, such as respiratory disorder, heart disease, diabetes, and premature birth, develop when the periodontal disease has become chronic.

An antimicrobial agent including as an active ingredient a cocoa fraction contained in cacao mass is known as an antimicrobial agent for periodontal bacteria (refer to Patent Document 1). However, in the production of the antimicrobial agent including the cocoa fraction as an active ingredient, troublesome pretreatment, i.e., roasting of cacao beans, is necessary to obtain cacao mass as a raw material.

At the same time, as described in Patent Document 2, it is known that an extract of *Macaranga tanarius* (Oobagi), which belongs to the genus *Macaranga* of the family *Euphorbiaceae,* has an antimicrobial action. However, the action of the *Macaranga tanarius* extract on periodontal bacteria has not been clarified yet, and also Patent Document 2 does not describe it at all.
Patent Document 1: International Publication No. WO 2003/99304
Patent Document 2: Japanese Laid-Open Patent Publication No. 2007-45754

### DISCLOSURE OF THE INVENTION

The present invention is based on the fact that the inventors have found, as a result of their intensive studies, that an extract of *Macaranga tanarius* has an inhibitory action on the growth of periodontal bacteria, and an objective thereof is to provide a periodontal bacterial growth inhibitor that can be produced without troublesome pretreatment of raw material and to provide an oral hygiene product and a food and drink that contain the inhibitor.

In order to achieve the above-mentioned objective, a first aspect of the present invention provides a periodontal bacterial growth inhibitor containing as an active ingredient a *Macaranga tanarius* extract extracted from *Macaranga tanarius* with an extraction solvent including at least an organic solvent.

A second aspect of the present invention provides a periodontal bacterial growth inhibitor containing as an active ingredient at least one selected from nymphaeol-A, nymphaeol-B, and nymphaeol-C.

A third aspect of the present invention provides an oral hygiene product containing the periodontal bacterial growth inhibitor according to the first or the second aspect.

A fourth aspect of the present invention provides a food and drink containing the periodontal bacterial growth inhibitor according to the first or the second aspect.

### BRIEF DESCRIPTION OF THE DRAWINGS

Fig. 1 is a chromatogram showing the results of high-performance liquid chromatography analysis of an extract of *Macaranga tanarius* according to Example 1; and
Fig. 2 is a chromatogram showing the results of high-performance liquid chromatography analysis of an extract of *Macaranga tanarius* according to Example 2.

### BEST MODE FOR CARRYING OUT THE INVENTION

An embodiment of the present invention will be described in detail below.

A periodontal bacterial growth inhibitor of the present embodiment contains as an active ingredient an Oobagi extract extracted from Oobagi with an extraction solvent including at least an organic solvent. Oobagi is also called *Macaranga tanarius* and is a dioecious broad-leaved evergreen tree belonging to the genus *Macaranga* of the family *Euphorbiaceae. Macaranga tanarius* grows, for example, in Southeast Asia, such as Okinawa (southern Japan), Taiwan, southern China, the Malay Peninsula, the Philippines, Malaysia, Indonesia, and Thailand, and in northern Australia. *Macaranga tanarius* grows significantly fast compared to other trees and can grow on degraded lands.

All the organs of *Macaranga tanarius* and constituents of each organ can be used as raw material to be subjected to extraction with the extraction solvent. The raw material for extraction may be a single organ of *Macaranga tanarius* or its constituents or may be a mixture of two or more organs of *Macaranga tanarius* or their constituents. In order to enhance the growth-inhibitory action of the resulting *Macaranga tanarius* extract on periodontal bacteria, it is preferred to use the raw material for extraction which includes fruit, seeds, flowers, roots, a trunk, the tip of a stem, a leaf blade, or an exudate (such as wax) of *Macaranga tanarius.* Since the tip of the stem includes a growth point of the stem and a leaf bud and is softer than the leaf blade, an efficient extraction procedure thereof is easy. Furthermore, the occupation ratios of the trunk, the roots, and the leaves to the entire *Macaranga tanarius* are high compared to those of other organs. Therefore, the use of leaf blade of *Macaranga tanarius,* which is easy to handle, as a raw material for extraction is industrially advantageous from the standpoint of easiness of obtaining the raw material.

The raw material for extraction is subjected to an extraction procedure in the state when it is harvested, in the state that it is pulverized, crushed, or ground after the harvest, in the state that it is pulverized, crushed, or ground after the harvest and drying, or in the state that it is pulverized, crushed, or ground after the harvest and then is dried. In order to efficiently perform the extraction, the raw material for extraction is preferably crushed. The crushing of the raw material for extraction can be performed, for example, using a cutter, a shredder, or a crusher. The shape of the raw material for extraction after the crushing is not particularly limited, and may be, for example, polygonal such as triangular or quadrangular. When the raw material for extraction after the crushing has a polygonal shape, each side is preferably about 1 cm long. The raw material for extraction can be pulverized using, for example, a mill, a crusher, or a grinder. The raw material for extraction can be ground using, for example, a kneader or a mortar.

The extraction solvent used for extracting a *Macaranga tanarius* extract from the raw material for extraction may be a solvent mixture of water and an organic solvent or may be an organic solvent such as lower alcohol, dimethyl sulfoxide, acetonitrile, acetone, ethyl acetate, hexane, glycerin, or propylene glycol. Examples of the lower alcohol that can be used include methanol, ethanol, propanol, isopropanol, and butanol. As the organic solvent, only one type of solvent may be used, or a mixture of a plurality of types of solvents may be used. Some oral hygiene products, such as dentifrices and mouthwashes, contain glycerin or propylene glycol as, for example, a solvent or a moisturizer. Accordingly, when a *Macaranga tanarius* extract solution obtained using glycerin or propylene glycol as the extraction solvent is blended to an oral hygiene product, the further blending of glycerin or propylene glycol as, for example, a solvent or a moisturizer to the oral hygiene product can be omitted. Therefore, in a *Macaranga tanarius* extract solution that is used by being blended to an oral hygiene product, the extraction preferably uses a solvent including glycerin or propylene glycol. When a solvent mixture of water and an organic solvent is used as the extraction solvent, the content of the organic solvent in the solvent mixture is preferably 50% by volume or more and more preferably 80% by volume or more. When the content of the organic solvent in a solvent mixture is 50% by volume or more, the active ingredient contained in *Macaranga tanarius* can be particularly efficiently extracted. The organic solvent is preferably lower alcohol and more preferably ethanol.

In the extraction solvent, an organic salt, an inorganic salt, a buffer, an emulsifier, dextrin, and so on may be dissolved.

The extraction is performed by immersing the raw material for extraction in the extraction solvent for a predetermined time. In the extraction, according to need, for example, either stirring or heating or the both of them may be conducted for increasing the extraction efficiency. Furthermore, in order to minimize extraction of unnecessary impurities into the extraction solvent, prior to the extraction with the extraction solvent, the raw material for extraction may be prepared by being subjected to extraction with water or hot water and removing the extraction water in advance. The ingredient that is contained in *Macaranga tanarius* and presumably has an inhibitory action on the growth of periodontal bacteria is nymphaeols. The nymphaeols are water-insoluble. Impurities other than the nymphaeols are efficiently transferred to extraction water by boiling *Macaranga tanarius* with, for example, hot water and are thereby removed.

A *Macaranga tanarius* extract extracted from the raw material for extraction is subjected to solid liquid separation to separate and remove the residue of the raw material for extraction. The solid liquid separation is performed, for example, by a known method such as filtration or centrifugation. The *Macaranga tanarius* extract in a liquid form after the solid liquid separation may be concentrated according to need.

A *Macaranga tanarius* extract in a solid form can be obtained by removing the extraction solvent contained in the *Macaranga tanarius* extract in the liquid form, according to need. The removal of the extraction solvent from the *Macaranga tanarius* extract in the liquid form may be performed, for example, by heating under reduced pressure or by lyophilization.

The *Macaranga tanarius* extract extracted from *Macaranga tanarius* with an extraction solvent including at least an organic solvent contains at least one selected from nymphaeol-A (also known as 5,7,3',4'-tetrahydroxy-6-geranylflavanone), nymphaeol-B (also known as 5,7,3',4'-tetrahydroxy-2'-geranylflavanone), and nymphaeol-C (also known as 5,7,3',4'-tetrahydroxy-6-(3"',3"'-dimethylallyl)-2'-geranylflavanone). A main ingredient of the *Macaranga tanarius* extract is at least one selected from nymphaeol-A, nymphaeol-B, and nymphaeol-C, that is, nymphaeols, and the nymphaeols presumably have an inhibitory action on the growth of periodontal bacteria.

The *Macaranga tanarius* extract further contains propolin A (also known as 5,7,3',4'-tetrahydroxy-2'-(7"-hydroxy-3",7"-dimethyl-2"-octenyl)-flavanone). Furthermore, the *Macaranga tanarius* extract contains as minor ingredients, for example, 5,7,3',4'-tetrahydroxy-5'-geranylflavanone (also known as isonymphaeol-B), 5,7,3',4'-tetrahydroxy-5'-(7"-hydroxy-3",7"-dimethyl-2"-octenyl)-flavanone, 5,7,3',4'-tetrahydroxy-6-(7"-hydroxy-3",7"-dimethyl-2"-octenyl)-flavanone, 5,7,4'-trihydroxy-3'-(7"-hydroxy-3",7"-dimethyl-2"-octenyl)-flavanone, and 5,7,4'-trihydroxy-3'-geranyiflavanone.

Among extract solutions each extracted from portions of *Macaranga tanarius,* an extract solution extracted from seeds containing wax particularly contains high concentrations of nymphaeol-A, B, and C and isonymphaeol-B.

The periodontal bacterial growth inhibitor may contain a component other than the *Macaranga tanarius* extract as long as the inhibitory action on the growth of periodontal bacteria is not impaired. Examples of the component that can be contained in the periodontal bacterial growth inhibitor, in addition to the *Macaranga tanarius* extract, include an excipient, a base, an emulsifier, a stabilizer, a flavoring, and a sweetening.

The periodontal bacterial growth inhibitor may be in a liquid form or in a solid form. The dosage form of the periodontal bacterial growth inhibitor is not particularly limited and may be, for example, a powder, a dust, a granule, a tablet, a capsule, a pill, a liquid, or an injection.

Periodontal disease developed by periodontal bacterial infection is roughly classified into gingivitis, periodontitis, and occlusal trauma. The periodontal bacterial growth inhibitor of the present embodiment inhibits the growth of periodontal bacteria and is thereby used for treatment and prevention of the periodontal disease.

As the periodontal bacteria, bacteria belonging to the genus *Actinobacillus,* the genus *Porphyromonas,* the genus *Prevotella,* or the genus *Fusobacterium* are known. Examples of the periodontal bacteria belonging to the genus *Actinobacillus* include *Actinobacillus actinomycetemcomitans.* Examples of the periodontal bacteria belonging to the genus *Porphyromonas* include *Porphyromonas gingivalis, Porphyromonas asaccharolytica,* and *Porphyromonas endodontalis.* Examples of the periodontal bacteria belonging to the genus *Prevotella* include *Prevotella intermedia, Prevotella nigrescens,* and *Prevotella melaninogenica.* Examples of the periodontal bacteria belonging to the genus *Fusobacterium* include *Fusobacterium nucleatum, Fusobacterium necrophorum,* and *Fusobacterium naviforme* AA.

Among these periodontal bacteria, the periodontal bacteria belonging to the genus *Actinobacillus* and the periodontal bacteria belonging to the genus *Porphyromonas* are the main causative bacteria of the periodontal disease. The periodontal bacterial growth inhibitor of the present embodiment exerts excellent effects in preventing and treating periodontal disease by inhibiting the growth of at least either the periodontal bacteria belonging to the genus *Actinobacillus* or the periodontal bacteria belonging to the genus *Porphyromonas.*

The periodontal bacterial growth inhibitor is utilized as, for example, an oral hygiene product, a food and drink, a drug, and a quasi drug. From the viewpoints that the active ingredient is easily brought into contact with periodontal tissue and that a sufficient action of the active ingredient is easily accomplished, the periodontal bacterial growth inhibitor is preferably used as an oral hygiene product or a food and drink.

An oral hygiene product of the present embodiment contains the above-mentioned periodontal bacterial growth inhibitor. Examples of the oral hygiene product include tooth powders, toothpastes, liquid dentifrices, mouthwashes, gingival massage creams, local liniments, troches, chewing gums, and dental flosses. Since the oral hygiene product containing the periodontal bacterial growth inhibitor can inhibit the growth of periodontal bacteria in the oral cavity, it is effective for prevention and treatment of periodontal disease. The oral hygiene product preferably contains the *Macaranga tanarius* extract in the range of 0.001 to 10% by mass, more preferably 0.01 to 1% by mass, in terms of solid content. When the content of the *Macaranga tanarius* extract contained in the oral hygiene product is 0.001 % by mass or more in terms of solid content, the growth of periodontal bacteria is particularly effectively inhibited by the oral hygiene product. However, when the content of the *Macaranga tanarius* extract in the oral hygiene product is higher than 10% by mass in terms of solid content, a high growth-inhibitory effect on periodontal bacteria proportional to the content is not achieved. The oral hygiene product may contain, for example, an emulsifier, a solvent, and a stabilizer, according to need, in addition to the base corresponding to the form.

A food and drink (beverage and food) of the present embodiment contains the above-described periodontal bacterial growth inhibitor. Examples of the beverage include carbonated drinks, tea drinks, cooling drinks, alcoholic drinks, milk, coffee, fruit syrups, jerry drinks, and energy drinks. The carbonated drink may be, for example, soda pop, lemon soda, or cola. The tea drink may be, for example, green tea, oolong tea, or black tea. The cooling drink may be, for example, fruit juice drink or mineral water. The alcoholic drink may be, for example, beer, low-molt beer, sake, whiskey, shochu, or cocktail. Examples of the food include yogurt, soup, curry, throat lozenges, candy, cookies, cake, Japanese confectionery, snacks, and syrups.

The food and drink may be, for example, health food, food for specified health use, health drink, or dietary supplements. The food and drink preferably contains the *Macaranga tanarius* extract in the range of 0.001 to 10% by mass, more preferably 0.01 to 1% by mass, in terms of solid content. When the content of the *Macaranga tanarius* extract contained in the food and drink is 0.001 % by mass or more in terms of solid content, the growth of periodontal bacteria is particularly effectively inhibited by the food and drink. However, when the content of the *Macaranga tanarius* extract in the food and drink is higher than 10% by mass in terms of solid content, a high growth-inhibitory effect on periodontal bacteria proportional to the content is not achieved. The food and drink may contain, for example, an emulsifier, a solvent, and a stabilizer, according to need, in addition to the base corresponding to the form.

According to the present embodiment, the following advantageous effects are achieved.

The periodontal bacterial growth inhibitor of the present embodiment contains the extract of *Macaranga tanarius* as an active ingredient. The *Macaranga tanarius* extract can be easily and efficiently obtained by subjecting *Macaranga tanarius* to extraction with an extraction solvent including at least an organic solvent. That is, the periodontal bacterial growth inhibitor of the present embodiment can be produced without troublesome pretreatment of raw material.

In addition, since *Macaranga tanarius* grows significantly fast compared to other trees and can grow on degraded lands, the cultivation does not take much effort. Furthermore, since the *Macaranga tanarius* extract is originated from a plant, it is highly safe. Therefore, the periodontal bacterial growth inhibitor of the present embodiment is also excellent in stable supply of raw material, productivity, and safety.

The oral hygiene product and the food and drink of the present embodiment contain the above-described periodontal bacterial growth inhibitor and therefore have the same advantageous effects as those of the periodontal bacterial growth inhibitor.

The above-described embodiment may be modified as follows.

The periodontal bacterial growth inhibitor may contain at least one selected from nymphaeol-A, nymphaeol-B, and nymphaeol-C that are not originated from *Macaranga tanarius* extracts, such as propolis from Okinawa, as an active ingredient, instead of the *Macaranga tanarius* extract or in addition to the *Macaranga tanarius* extract.

Next, the present invention will be further specifically described with reference to examples.

### Example 1

### <Preparation 1 of Macaranga tanarius extract>

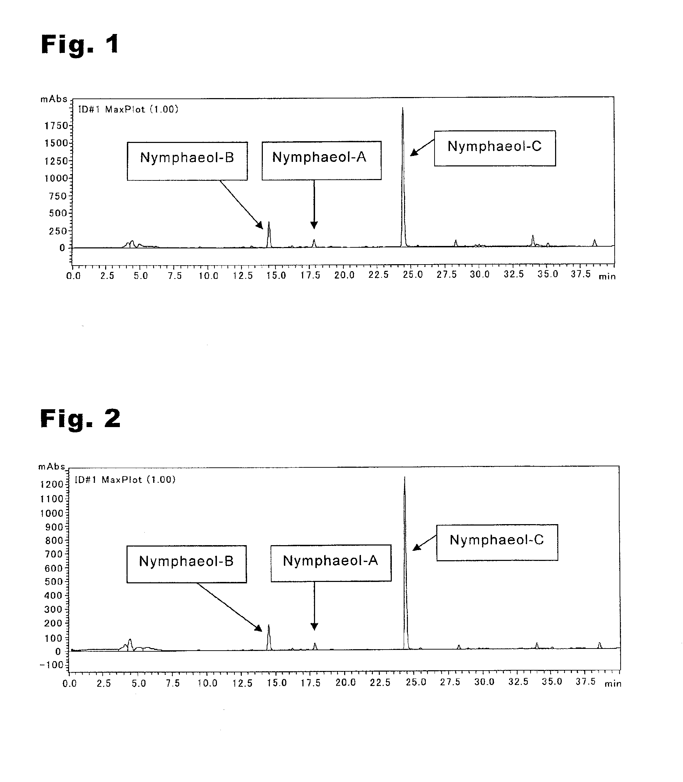

Frozen raw leaves of *Macaranga tanarius* harvested in Okinawa were thawed, and the leaves were cut into small pieces with scissors. Thirty grams of the cut raw leaves were immersed in 100 mL of a solvent mixture consisting of 90 parts by volume of ethanol and 10 parts by volume of water and left standing at room temperature for two weeks, followed by filtration to yield the filtrate as a *Macaranga tanarius* extract solution. The *Macaranga tanarius* extract solution was lyophilized to prepare a *Macaranga tanarius* extract that was a powder of the solid content contained in the *Macaranga tanarius* extract solution. The total concentration of nymphaeol-A, nymphaeol-B, and nymphaeol-C, that is, the concentration of nymphaeols, in the *Macaranga tanarius* extract in the powder form was 50% by mass when calculated from the chromatogram shown in Fig. 1 obtained by analyzing the *Macaranga tanarius* extract under the following HPLC conditions.

### HPLC conditions

System: PDA-HPLC system (Shimadzu Corp.), LC10ADvp series, UV: SPD-10Avp, PDA: SPD-M10Avp,
Column: Luna C18 (2.0 x 250 mm) (Shimadzu GLC),
Solvent: A: water (5% acetic acid), B: acetonitrile (5% acetic acid)

### Dissolution condition:

0 to 20 minutes
(gradient dissolution : A:B = 80:20 → A:B = 30:70)
20 to 50 minutes
(gradient dissolution : A:B = 30:70 → A:B = 0:100)
50 to 60 minutes (A:B = 0:100)
60 to 75 minutes (A:B = 80:20)
   Flow rate: 0.2 mL/min
   PDA detection: UV from 190 to 370 nm
   UV detection: UV 287 nm
   Injection amount: 20 µL
   Temperature: 40°C

### <Test of antimicrobial activity of Macaranga tanarius extract on periodontal bacteria>

A periodontal bacterial strain 1 (*Actinobacillus actinomycetemcomitans* (JCM8577)) and a periodontal bacterial stain 2 (*Porphyromonas gingivalis* (JCM12257)) were each inoculated on a GAM agar medium with a platinum loop and were cultured at 35°C for 7 days.

The grown colonies of periodontal bacterial strain 1 by the culturing were harvested from the GAM agar medium with a platinum loop and were dissolved in physiological saline to prepare a 10⁴ cfu/mL of bacterial inoculum solution 1. The grown colonies of periodontal bacterial strain 2 by the culturing were harvested from the GAM agar medium with a platinum loop and were dissolved in physiological saline to prepare a 10⁴ cfu/mL of bacterial inoculum solution 2.

The *Macaranga tanarius* extract obtained in the "Preparation 1 of *Macaranga tanarius* extract" above was mixed with a GAM agar medium to prepare a plate medium. The final concentration of the *Macaranga tanarius* extract was adjusted to 100 ppm to give a sample plate medium 1, the final concentration of the *Macaranga tanarius* extract was adjusted to 250 ppm to give a sample plate medium 2, and the final concentration of the *Macaranga tanarius* extract was adjusted to 500 ppm to give a sample plate medium 3. Separately, ethanol was mixed with a GAM agar medium, and the final concentration of the ethanol was adjusted to 0.7% to give a blank plate medium.

The sample plate media 1 to 3 and the blank plate medium were each sterilized under conditions of 115°C for 15 minutes. The bacterial inoculum solution 1 and the bacterial inoculum solution 2 were each smeared on each of the plate media with a platinum loop. Then, the periodontal bacterial strain 1 and the periodontal bacterial strain 2 on each plate medium were cultured at 35°C for 7 days using an anaerobic system manufactured by Mitsubishi Gas Chemical Co.

After the culturing, the presence or absence of colonies on the plate media was checked. Regarding the periodontal bacterial strain 1, colonies were observed on the blank plate medium and the sample plate medium 1, but no colonies were observed on the sample plate media 2 and 3. Furthermore, regarding the periodontal bacterial strain 2, colonies were observed on the blank plate medium, but no colonies were observed on the sample plate media 1 to 3. That is, it was confirmed that the growth of the periodontal bacterial strain 1 is inhibited when the concentration of the *Macaranga tanarius* extract is 250 ppm or more (125 ppm or more in terms of nymphaeol concentration) and that the growth of the periodontal bacterial strain 2 is inhibited when the concentration of the *Macaranga tanarius* extract is 100 ppm or more (50 ppm or more in terms of nymphaeol concentration). The results have revealed that the growth-inhibitory effect of the *Macaranga tanarius* extract of this Example on the periodontal bacterial strain 2 is higher than that on the periodontal bacterial strain 1.

### Example 2

### <Preparation 2 of Macaranga tanarius extract>

Thirty grams of cut raw leaves of *Macaranga tanarius* were immersed in 95°C water for 30 minutes. The water was removed by filtration, and the remaining leaves were immersed in 100% ethanol for 3 days, followed by filtration to yield the filtrate as a *Macaranga tanarius* extract solution. The *Macaranga tanarius* extract solution was lyophilized to prepare a *Macaranga tanarius* extract that was a powder of the solid content contained in the *Macaranga tanarius* extract solution. The total concentration of nymphaeol-A, nymphaeol-B, and nymphaeol-C, that is, the concentration of nymphaeols, in the *Macaranga tanarius* extract in the powder form was 40% by mass when calculated from the chromatogram shown in Fig. 2 obtained by analyzing the *Macaranga tanarius* extract solution under the above-mentioned HPLC conditions.

### <Test of antimicrobial activity of Macaranga tanarius extract on periodontal bacteria>

An antimicrobial activity test was performed as in Example 1. Regarding the *Macaranga tanarius* extract prepared in Example 2, similar results as those of the *Macaranga tanarius* extract prepared in Example 1 were obtained to confirm that the antimicrobial activities of the *Macaranga tanarius* extract prepared in Example 1 and the *Macaranga tanarius* extract prepared in Example 2 were similar to each other.

## Claims

1. A periodontal bacterial growth inhibitor **characterized by** comprising as an active ingredient a *Macaranga tanarius* extract extracted from *Macaranga tanarius* with an extraction solvent including at least an organic solvent.

2. A periodontal bacterial growth inhibitor **characterized by** comprising as an active ingredient at least one selected from nymphaeol-A, nymphaeol-B, and nymphaeol-C.

3. The periodontal bacterial growth inhibitor according to claim 2, wherein the at least one selected from nymphaeol-A, nymphaeol-B, and nymphaeol-C is originated from an extract of *Macaranga tanarius.*

4. The periodontal bacterial growth inhibitor according to any one of claims 1 to 3, wherein the inhibitor has an inhibitory action on the growth of at least either periodontal bacteria belonging to the genus *Actinobacillus* or periodontal bacteria belonging to the genus *Porphyromonas.*

5. An oral hygiene product **characterized by** comprising the periodontal bacterial growth inhibitor according to any one of claims 1 to 4.

6. A food and drink **characterized by** comprising the periodontal bacterial growth inhibitor according to any one of claims 1 to 4.
